# EUROPEAN PATENT SPECIFICATION

(11) **EP 1 276 458 B1**
(45) Date of publication and mention of the grant of the patent: **27.09.2006**
(21) Application number: 01923849.2
(22) Date of filing: 27.04.2001
(51) Int. Cl.: A61Q 19/00, A61K 8/92

(54) **COSMETIC LOTIONS COMPRISING COCOA BUTTER**
KOSMETISCHE ZUBEREITUNGEN KAUKAUBUTTER ENTHALTEND
LOTIONS COSMETIQUES RENFERMANT DU BEURRE DE CACAO

(30) Priority: 28.04.2000 GB 0010425
(43) Date of publication of application: 22.01.2003
(73) Proprietor: Lush Limited, Dorset BH15 1AB (GB)
(72) Inventor: AMBROSEN, Helen, Poole, Dorset BH15 1AB (GB); CONSTANTINE, Mark, Poole, Dorset BH15 1AB (GB); CONSTANTINE, Margaret, Poole, Dorset BH15 1AB (GB); BIRD, Rowena Jacqueline, Bournemouth BH4 9JA (GB)
(74) Representative: Sorenti, Gino
(86) International application number: PCT/GB2001/001870
(87) International publication number: WO 2001/082889

(56) References cited:
- DE-A- 3 246 265
- DE-U- 29 810 730
- FR-A- 918 615
- GB-A- 1 298 154
- US-A- 3 499 446
- US-A- 5 997 889

## Description

The present invention relates to cosmetic lotions.

Cosmetic lotions are useful preparations for softening and moisturising the skin. They are commonly made from oil based ingredients and water based ingredients held together in suspension by an emulsifier. Without an emulsifier the oil and water phases would separate. Lotions are flowing, liquid preparations. They are normally packaged in a rigid container made of glass or plastic which is often non biodegradable.

According to a first aspect of the present invention there is provided a cosmetic lotion comprising water based component being

| | |
|---|---|
| Glycerine, Water or Water Based Plant Extract | 13.0% - 2.0% |
| Honey, Fresh Fruit or Vegetable | 60.0% - 20.0% |
| Fragrance | 0.5% 1.0% |

and an oil based component including cocoa butter, the components and any additions constituting 100% by weight of the final form of the lotion with the water based component being by weight in the range of 23% to 73.5% and with the cocoa butter constituting 16% to 76% by weight of the final form of the lotion, such that the final form of the lotion is a solid, such as a bar.

According to a second aspect of the present invention there is provided a method of manufacturing a cosmetic lotion having an oil based component and a water based component with these components and any additions constituting 100% by weight of the final form of the lotion characterised by selecting the water based component which is composed of

| | |
|---|---|
| Glycerine, Water or Water Based Plant Extract | 13.0% - 2.0% |
| Honey, Fresh Fruit or Vegetable | 60.0% - 20.0% |
| Fragrance | 0.5% 1.0% |

to constitute 23% to 73.5% by weight of the final form of the lotion and by selecting the oil based component to comprise cocoa butter so that the cocoa butter constitutes 16% to 76% by weight of the final form of the lotion; heating the cocoa butter to a temperature in the range 55° to 70° C; cooling the cocoa butter to a temperature in the range 35° to 25° C and at a temperature in that range adding the water based component, whereby the final form of the lotion is a solid such as a bar.

Embodiments of the present invention will now be described, by way of example only.

The present invention utilises the emulsifying properties of cocoa butter to form a novel emulsion system. It contains water based and oil based ingredients. These would normally separate into two phases. Using Cocoa Butter as an emulsifier holds the ingredients in suspension. The nature of Cocoa Butter is a solid and this gives the structure of a bar.

Cocoa Butter comes from the bean of the Cocao tree (Theobroma cacao), more commonly known as cocoa beans. The beans are fermented, roasted and ground to produce an oily paste. The cocoa fat, more than 50% of the bean, is rendered to produce cocoa butter. The remaining fat free, powered residue of the bean is cocoa.

In cosmetic products the emollient qualities of cocoa butter are used to soften the skin. However, the present invention is based on it's emulsifying qualities. Oil and water based ingredients are held together in suspension in the form of a bar. When applied to the skin the effect is the same as that of a lotion. Water based materials will moisturise and oil based ingredients will soften. After application, the skin feels moisturised but not heavily oiled as it would do if an oil-based cocoa butter bar had been applied.

The nature of Cocoa Butter is a solid and this forms the structure of the bar with the emulsion in situ. Many shapes and applications can be achieved because of this solid form.

The Solid Lotion Bar can be given further applications. By the addition of sunscreens it is capable of protecting the skin from the sun. The addition of harder waxes such as beeswax modifies the melting point. The addition of a material such as sodium bicarbonate provides a deodorising product. The addition of clays such as talc gives a product capable of absorbing excess moisture on the skin or solid 'talc'. Varying ingredients can be added to give formulae suitable for different skin types. Fruits and vegetables are compatible with the system. Their water based ingredients give the bar different applications, eg. Banana softens the skin, Garlic prevents the formation of spots on the skin.

An example of a cosmetic lotion according to the present invention is as follows:-

### EXAMPLE OF A LOTION IN SOLID BAR FORM

| Oil Based Ingredients | % |
|---|---|
| Cocoa Butter | 70.0 |
| Almond Oil | 2.0 |

| Water Based Ingredients | |
|---|---|
| Fresh Banana or Avocado | 21.5 |
| Plant extract eg. Water based extract of Aloe Vera | 5.5 |
| Fragrance | 1.0 |
| | 100.0 |

According to the present invention the cocoa butter should constitute between 16% to 76% (inclusive) by weight of the final product. Preferably the cocoa butter should constitute between 30% to 75% (inclusive) by weight of the final product, more preferably 50 % to 75% and most preferably 60 % to 75%. Generally, it has been found that the advantages of the invention can be obtained using the following percentage ranges (by weight) of various desirable ingredients, namely:-

### RANGE OF PERCENTAGES FOR INGREDIENTS IN SOLID LOTION BAR

| Oil Based Ingredients | % | % |
|---|---|---|
| Cocoa Butter | 16.0 - | 76.0 |
| Oil e.g. Almond | 10.5 - | 1.0 |

| Water Based Ingredients | | |
|---|---|---|
| Glycerine, Water or Water Based Plant Extract | 13.0 - | 2.0 |
| Honey, Fresh Fruit or Vegetable | 60.0 - | 20.0 |
| | | |
| Fragrance | 0.5 | 1.0 |
| | 100.0 | 100.0 |

The manufacturing process of the bar is also of consequence. The Cocoa Butter and oils are melted to 55°-70°C then cooled to 60°-25°C. The water based ingredients and fragrance are preferably added between 35° and 25°C. The mixture is then cooled to 30°-20°C and poured into moulds. These are chilled to 20°-10°C and hardened.

Heating Cocoa Butter to approximately 60°C prevents undesirable crystallisation on cooling. Emulsifying the water based ingredients at approximately 30°C ensures the emulsion forms fully. Cooling to approximately 25°C ensures the bars exit the moulds easily when formed. Chilling at 16°C allows the bars to cool quickly without cracking. Any substantial variation from these temperatures, at least with respect to the initial heating and the emulsifying stage, can result in a faulty final product.

As the bars are solid forms then can be packaged in biodegradable materials such as paper or cardboard. The bars can be treated by dipping in a molten hard wax such as beeswax or Japan wax to keep the hands clean when applying the bar to the body.

## Claims

1. A cosmetic lotion comprising water based component and an oil based component including cocoa butter, the components and any additions constituting 100% by weight of the final form of the lotion with the water based component being
| | |
|---|---|
| Glycerine, Water or Water Based Plant Extract | 13.0% - 2.0% |
| Honey, Fresh Fruit or Vegetable | 60.0% - 20.0% |
| Fragrance | 0.5% 1.0% |
being by weight in the range of 23% to 73.5% and with the cocoa butter constituting 16% to 76% by weight of the final form of the lotion, such that the final form of the lotion is a solid, such as a bar.

2. A cosmetic lotion as claimed in claim 1, comprising 30% to 75% by weight cocoa butter.

3. A cosmetic lotion as claimed in claim 1, comprising 50% to 75% by weight cocoa butter.

4. A cosmetic lotion as claimed in claim 1, comprising 60% to 75% by weight cocoa butter.

5. A cosmetic lotion as claimed in any preceding claim, wherein the lotion contains an oil based ingredient in addition to the cocoa butter.

6. A cosmetic lotion as claimed in claim 5, wherein the said oil based ingredient is almond oil.

7. A cosmetic lotion as claimed in any preceding claim, wherein the lotion contains a water based ingredient comprising part of a fruit.

8. A cosmetic lotion as claimed in any preceding claim, wherein the lotion contains a water based ingredient comprising part of a vegetable.

9. A cosmetic lotion as claimed in claim 1, comprising the following components totalling 100% by weight and each being in the following range by weight of the total:
| | |
|---|---|
| Cocoa Butter | 16.0% - 76.0% |
| Oil | 10.5% - 1.0% |
| Glycerine, Water or Water Based Plant Extract | 13.0% - 2.0% |
| Honey, Fresh Fruit or Vegetable | 60.0% - 20.0% |
| Fragrance | 0.5% 1.0% |

10. A cosmetic lotion as claimed in claim 1, comprising
| | (by weight) % |
|---|---|
| Cocoa Butter | 70.0 |
| Almond Oil | 2.0 |
| Fresh Banana or Avocado | 21.5 |
| Plant extract | 5.5 |
| Fragrance | 1.0 |

11. A method of manufacturing a cosmetic lotion having an oil based component and a water based component with these components and any additions constituting 100% by weight of the final form of the lotion **characterised by** selecting the water based component which is composed of
| | |
|---|---|
| Glycerine, Water or Water Based Plant Extract | 13.0% - 2.0% |
| Honey, Fresh Fruit or Vegetable | 60.0% - 20.0% |
| Fragrance | 0.5% 1.0% |
to constitute 23% to 73.5% by weight of the final form of the lotion and by selecting the oil based component to comprise cocoa butter so that the cocoa butter constitutes 16% to 76% by weight of the final form of the lotion; heating the cocoa butter to a temperature in the range 55° to 70° C; cooling the cocoa butter to a temperature in the range 35° to 25° C and at a temperature in that range adding the water based component, whereby the final form of the lotion is a solid such as a bar.

12. A method as claimed in claim 11, further comprising the step of: at a temperature in the range 30° to 20°C pouring the mixed oil and water based components in to one or more moulds.

13. A method as claimed in claim 12, further comprising the step subsequent to said step of pouring of chilling the lotion to a temperature in the range 20° to 10°C.

14. A method as claimed in claim 11, comprising the steps of: heating the Cocoa Butter to approximately 60°C, emulsifying the water based ingredient at approximately 30°C, cooling the lotion to approximately 25°C, pouring the lotion in to one or more moulds and chilling the lotion at 16°C.

## Patentansprüche

1. Kosmetische Lotion umfassend einen wasserhaltigen Bestandteil und einen ölhaltigen Bestandteil, der Kakaobutter enthält, wobei die Bestandteile und jegliche Zusatzstoffe 100 Gew.-% der endgültigen Form der Lotion ausmachen, wobei der wasserhaltige Bestandteil
| | |
|---|---|
| Glycerin, Wasser oder wasserhaltiges Pflanzenextrakt | 13,0 Gew.-% - 2,0 Gew.-% |
| Honig, Frischobst oder Gemüse | 60,0 Gew.-% - 20,0 Gew.-% |
| Duftstoff | 0,5 Gew.-% - 1,0 Gew.-% |
im Bereich von 23 Gew.-% bis 73,5 Gew.-% und die Kakaobutter 16 Gew.-% bis 76 Gew.-% der endgültigen Form der Lotion ausmachen, und wobei die endgültige Form der Lotion ein Feststoff, zum Beispiel eine Stange, ist.

2. Kosmetische Lotion nach Anspruch 1, die 30 Gew.-% bis 75 Gew.-% Kakaobutter umfasst.

3. Kosmetische Lotion nach Anspruch 1, die 50 Gew.-% bis 75 Gew.-% Kakaobutter umfasst.

4. Kosmetische Lotion nach Anspruch 1, die 60 Gew.-% bis 75 Gew.-% Kakaobutter umfasst.

5. Kosmetische Lotion nach einem der vorhergehenden Ansprüche, wobei die Lotion zusätzlich zur Kakaobutter einen ölhaltigen Bestandteil enthält.

6. Kosmetische Lotion nach Anspruch 5, wobei der ölhaltige Bestandteil Mandelöl ist.

7. Kosmetische Lotion nach einem der vorhergehenden Ansprüche, wobei die Lotion einen wasserhaltigen Bestandteil, der einen Teil einer Frucht umfasst, enthält.

8. Kosmetische Lotion nach einem der vorhergehenden Ansprüche, wobei die Lotion einen wasserhaltigen Bestandteil, der einen Teil eines Gemüses umfasst, enthält.

9. Kosmetische Lotion nach Anspruch 1, die folgenden, insgesamt 100 Gew.-% ausmachenden Bestandteile umfassend, wobei jeder Bestandteil im folgenden Gewichtsanteil vom Ganzen vorliegt:
| | |
|---|---|
| Kakaobutter | 16,0 - 70,0 |
| Öl, z. B. Mandelöl | 10,5 - 1, 0 |
| Glycerin, Wasser oder wasserhaltiges Pflanzenextrakt | 13,0 - 2,0 |
| Honig, Frischobst oder Gemüse | 60,0 - 20,0 |
| Duftstoff | 0,5 - 1,0 |

10. Kosmetische Lotion nach Anspruch 1, umfassend
| | Gew.-% |
|---|---|
| Kakaobutter | 70,0 |
| Mandelöl | 2,0 |
| Frische Banane oder Avocado | 21,5 |
| Pflanzenextrakt | 5,5 |
| Duftstoff | 1,0 |

11. Verfahren zur Herstellung einer kosmetischen Lotion, die einen ölhaltigen Bestandteil und einen wasserhaltigen Bestandteil umfasst, wobei diese Bestandteile und jegliche Zusatzstoffe 100 Gew.-% der endgültigen Form der Lotion ausmachen, **dadurch gekennzeichnet, dass** der wasserhaltige Bestandteil
| | |
|---|---|
| Glycerin, Wasser oder wasserhaltiges Pflanzenextrakt | 13,0 Gew.-% - 2,0 Gew.-% |
| Honig, Frischobst oder Gemüse | 60,0 Gew.-% - 20,0 Gew.-% |
| Duftstoff | 0,5 Gew.-% - 1,0 Gew.-% |
so gewählt wird, dass er 23 Gew.-% bis 75,5 Gew.-% der endgültigen Form der Lotion ausmacht, und der ölhaltige Bestandteil so gewählt wird, dass er Kakaobutter umfasst und dass die Kakaobutter 16 Gew.-% bis 76 Gew.-% der endgültigen Form der Lotion ausmacht; wobei die Kakaobutter auf eine Temperatur im Bereich von 55 °C bis 70 °C erhitzt wird, wobei die Kakaobutter auf eine Temperatur im Bereich von 35 °C bis 25 °C gekühlt wird; und dass bei einer Temperatur in diesem Bereich der wasserhaltige Bestandteil hinzugefügt wird, wobei die endgültige Form der Lotion ein Feststoff wie eine Stange ist.

12. Verfahren nach Anspruch 11, das außerdem den Schritt umfasst, dass bei einer Temperatur im Bereich von 30 °C bis 20 °C die miteinander vermischten öl- und wasserhaltigen Bestandteile in eine oder mehrere Formen gegossen werden.

13. Verfahren nach Anspruch 12, das außerdem im Anschluss an das Vergießen den Schritt umfasst, dass die Lotion auf eine Temperatur im Bereich von 20 °C bis 10 °C abgekühlt wird.

14. Verfahren nach Anspruch 11, umfassend die Schritte: Erhitzen der Kakaobutter auf ungefähr 60 °C, Emulgieren des wasserhaltigen Bestandteils bei ungefähr 30 °C, Kühlen der Lotion auf ungefähr 25 °C, Gießen der Lotion in eine oder mehrere Formen und Abkühlen der Lotion bei 16 °C.

## Revendications

1. Lotion cosmétique comprenant un composant à base d'eau et un composant à base d'huile comprenant du beurre de cacao, les composants et toutes les additions constituant 100 % en poids de la forme finale de la lotion avec le composant à base d'eau étant
| | |
|---|---|
| glycérine, eau ou extrait végétal à base d'eau | 13,0 % - 2,0 % |
| miel, fruit ou légume frais | 60,0 % - 20,0 % |
| parfum | 0,5 % - 1,0 % |
étant en poids dans la plage de 23 % à 73,5 % et avec le beurre de cacao constituant de 16 % à 76 % en poids de la forme finale de la lotion, de telle sorte que la forme finale de la lotion est un solide, tel qu'une barre.

2. Lotion cosmétique selon la revendication 1, comprenant de 30 % à 75 % en poids de beurre de cacao.

3. Lotion cosmétique selon la revendication 1, comprenant de 50 % à 75 % en poids de beurre de cacao.

4. Lotion cosmétique selon la revendication 1, comprenant de 60 % à 75 % en poids de beurre de cacao.

5. Lotion cosmétique selon l'une quelconque des revendications précédentes, dans laquelle la lotion contient un ingrédient à base d'huile outre le beurre de cacao.

6. Lotion cosmétique selon la revendication 5, dans laquelle ledit ingrédient à base d'huile est de l'huile d'amande douce.

7. Lotion cosmétique selon l'une quelconque des revendications précédentes, dans laquelle la lotion contient un ingrédient à base d'eau comprenant une partie d'un fruit.

8. Lotion cosmétique selon l'une quelconque des revendications précédentes, dans laquelle la lotion contient un ingrédient à base d'eau comprenant une partie d'un légume.

9. Lotion cosmétique selon la revendication 1, comprenant les composants suivants totalisant 100 % en poids et chacun étant dans la plage suivante en poids du total :
| | |
|---|---|
| beurre de cacao | 16,0 % - 76,0 % |
| huile | 10,5 % - 1,0 % |
| glycérine, eau ou extrait végétal à base d'eau | 13,0 % - 2,0 % |
| miel, fruit ou légume frais | 60,0 % - 20,0 % |
| parfum | 0,5 % - 1,0 % |

10. Lotion cosmétique selon la revendication 1, comprenant
| | % (en poids) |
|---|---|
| beurre de cacao | 70,0 |
| huile d'amande douce | 2,0 |
| banane fraîche ou avocat frais | 21,5 |
| extrait végétal | 5,5 |
| parfum | 1,0 |

11. Procédé de fabrication d'une lotion cosmétique ayant un composant à base d'huile et un composant à base d'eau avec ces composants et toutes les additions constituant 100 % en poids de la forme finale de la lotion, **caractérisé par** le choix du composant à base d'eau qui est composé de
| | |
|---|---|
| glycérine, eau ou extrait végétal à base d'eau | 13,0 % - 2,0 % |
| miel, fruit ou légume frais | 60,0 % - 20,0 % |
| parfum | 0,5 % - 1,0 % |
de manière à ce qu'il constitue de 23 % à 73,5 % en poids de la forme finale de la lotion et par le choix du composant à base d'huile pour qu'il comprenne du beurre de cacao de telle manière que le beurre de cacao constitue de 16 % à 76 % en poids de la forme finale de la lotion ; le chauffage du beurre de cacao à une température dans la plage de 55° à 70°C ; le refroidissement du beurre de cacao à une température dans la plage de 35° à 25°C et, à une température dans cette plage, l'addition du composant à base d'eau, moyennant quoi la forme finale de la lotion est un solide tel qu'une barre.

12. Procédé selon la revendication 11, comprenant en outre l'étape consistant à : à une température dans la plage de 30° à 20°C, verser les composants à base d'huile et à base d'eau mélangés dans un ou plusieurs moules.

13. Procédé selon la revendication 12, comprenant en outre l'étape, subséquente à ladite étape consistant à verser, consistant à refroidir la lotion à une température dans la plage de 20° à 10°C.

14. Procédé selon la revendication 11, comprenant les étapes consistant à : chauffer le beurre de cacao à approximativement 60°C, émulsifier l'ingrédient à base d'eau à approximativement 30°C, refroidir la lotion à approximativement 25°C, verser la lotion dans un ou plusieurs moules et refroidir la lotion à 16°C.
